# EUROPÄISCHE PATENTSCHRIFT

(11) **EP 2 800 594 B1**
(45) Veröffentlichungstag und Bekanntmachung des Hinweises auf die Patenterteilung: **04.01.2017**
(21) Anmeldenummer: 12797759.3
(22) Anmeldetag: 14.11.2012
(51) Int. Cl.: A61M 5/30

(54) **ZYLINDER-KOLBEN-EINHEIT MIT KLEBESCHEIBE II**
BARREL-PLUNGER UNIT WITH ADHESIVE DISK II
UNITÉ CYLINDRE-PISTON COMPORTANT UN DISQUE ADHÉSIF II

(30) Priorität: 16.11.2011 DE 102011119055
(43) Veröffentlichungstag der Anmeldung: 12.11.2014
(73) Patentinhaber: LTS Lohmann Therapie-Systeme AG, 56626 Andernach (DE)
(72) Erfinder: MATUSCH, Rudolf, 35041 Marburg (DE)
(74) Vertreter: Simandi, Claus
(86) Internationale Anmeldenummer: PCT/EP2012/072579
(87) Internationale Veröffentlichungsnummer: WO 2013/072347

(56) Entgegenhaltungen:
- WO-A1-2007/071485
- WO-A1-2007/088112
- WO-A2-2005/058393

## Beschreibung

Die Erfindung betrifft eine Zylinder-Kolben-Einheit, mit mindestens einem, eine Injektionslösung aufnehmenden Zylinder, mindestens einem Kolben und einer im Bereich der freien Stirnseite des Zylinders angeordneten Klebebeschichtung.

Aus der DE 10 2005 054 600 ist eine Zylinder-Kolben-Einheit eines nadelfreien Injektors bekannt, deren Zylinder an seiner vorderen Stirnseite eine mittels eines Haftklebers fixierte Dichtfolie aufweist. Der Haftkleber hat gegenüber der Zylinderstirnfläche eine größere Affinität als gegenüber der Dichtfolie, so dass er nach dem Abziehen der Dichtfolie dort zurückbleibt, um dann während der Injektion die Haut des Patienten gegenüber dem Injektor zu fixieren.

Die DE 698 36 594 T3 beschreibt eine Vorrichtung zum transkutanen Platzieren einer flexiblen Kanüle an einer Medikamenteneinführungsstelle am Patienten. Dazu besitzt die Vorrichtung einen Adaptersatz und einen spannbaren, in einem Gehäuse untergebrachten, Federspeicher. Der vorn in das Gehäuse einsetzbare Adaptersatz besteht im Wesentlichen aus einer Klebeplatte, einer flexiblen Kanüle und einer Einführungsnadel. Beim Auslösen der Vorrichtung wird der Adaptersatz mittels des sich entspannenden Federspeichers auf der Haut positioniert. Hierbei wird die Kanüle mit Hilfe der Einführungsnadel unter die Haut geschoben. Nach dem Entfernen des Gehäuses und dem Herausziehen der Einführungsnadel kann die Infusion beginnen.

Eine Klebescheibe gemäß der WO 2007/071485 A1 vermag nach Wegnahme der Zylinder-Kolben-Einheit nicht auf der Haut zurückzubleiben.

Bei nadelfreien Injektionen muss die Injektionslösung durch per Hochgeschwindigkeitsstrahl die Haut des Patienten appliziert werden. Die obere Hautschicht stellt eine widerstandsfähige mechanische Abschirmung der darunter liegenden Hautschichten gegenüber äußeren Einwirkungen dar. Sie besteht aus neben- und übereinander liegenden verhornten Zellen, deren Zwischenräume durch Lipide ausgekleidet sind.

Der vorliegenden Erfindung liegt daher die Problemstellung zugrunde, im Rahmen eines modular aufgebauten Injektors eine Zylinder-Kolben-Einheit zu entwickeln, deren Ausblassystem geeignet ist, die unter dem Begriff "Dermis" zusammengefassten äußeren Hautschichten oder je nach Aufgabe auch die restlichen sicher zu durchdringen, um die Injektionslösung in die Haut oder unter sie zu bringen und dort eine bestimmbare Dauer zu halten.

Diese Problemstellung wird mit den Merkmalen des Hauptanspruchs erfindungsgemäß gelöst. Dazu weist der Zylinder einen Bodenabschnitt auf, an dem ein Ausblasrohr angeordnet ist. Am Ausblasrohr und/oder am Bodenabschnitt ist eine in Richtung der Mittellinie der Zylinder-Kolben-Einheit zwischen einer Einbauposition und einer Applikationsposition verschiebbare, elastische Klebescheibe angeordnet. Die Klebescheibe weist auf ihrer dem Bodenabschnitt abgewandten Stirnseite eine Klebebeschichtung zur Verklebung mit der Haut auf. Die Klebescheibe weist in der Lagerposition mindestens ein Dichtelement oder einen Dichtbereich zum Verschließen des Ausblasrohres auf, dessen Wirkung in der Applikationsposition nicht mehr vorhanden ist. In der Applikationsposition steht die vordere Kante der freien Stirnseite des Ausblasrohres zur Eindellung der Haut - ohne diese zu schädigen - mindestens 0,5 mm über die Klebebeschichtung der Klebescheibe über. Durch das Aufdehnen der Klebescheibe und das Überstehen des Ausblasrohres ist die Haut spannbar. Die auf der Haut zurückbleibende Klebescheibe ist nach der Wegnahme der Zylinder-Kolben-Einheit separat entfernbar.

Mit der Erfindung wird hier beispielsweise die Zylinder-Kolben-Einheit eines nadelfreien Injektors vorgestellt. Der Injektor, der auch ein Einmalinjektor sein kann, lagert neben der Zylinder-Kolben-Einheit einen in einem Injektorgehäuse eingebauten, auf einen Kolbenbetätigungsstempel wirkenden Antrieb. Als mögliche Antriebe können Federspeicher, Gasantriebe mit öffenbaren Gaskartuschen oder pyrotechnische Antriebe verwendet werden. Bekannte Federenergiespeicher nutzen vorgespannte mechanische oder pneumatische Federn oder Federsysteme. Wird als Antrieb ein Federenergiespeicher benutzt, wird zum Vorspannen und Halten dieses Federenergiespeichers der Kolbenbetätigungsstempel über mindestens einen am oder im Injektorgehäuse angeordneten Stützstab oder Zughaken formschlüssig gehalten. Der oder die Stützstäbe bzw. Zughaken werden mittels eines oder mehrerer Auslöseelemente bis zum Gebrauch des Injektors in ihrer Sperrposition arretiert. Zum Auslösen des Injektors werden der oder die Stützstäbe bzw. Zughaken freigegeben, so dass sich der Kolbenbetätigungsstempel - unter der Wirkung des Federenergiespeichers - zumindest annähernd parallel zur Mittellinie des Injektors bewegen kann, um die im Zylinder der Zylinder-Kolben-Einheit vorhandene Injektionslösung über mindestens eine Düse auszustoßen.

Im vorliegenden Fall ist der Zylinder-Kolben-Einheit eine am Zylinder verschiebbare Klebescheibe vorgelagert. Beim Aufsetzen des einsatzbereiten Injektors auf die Haut des Patienten verklebt in einem ersten Schritt die Haut des Patienten mit der am Zylinder in einer Einbauposition sitzenden Klebescheibe. Die Klebescheibe hält in diesem Stadium nach wie vor den Zylinder verschlossen. Durch ein weiteres Andrücken des Injektors an die Haut verrutscht die Klebescheibe unter einem Aufstoßen des in der Klebescheibe integrierten Dichtmittels. Nach dem erwünschten Verschieben der Klebescheibe steht über diese ein am Zylinder angeformtes oder befestigtes Ausblasrohr über, das durch diesen Überstand die Haut im Bereich der Düse des Zylinders spannt.

Das Spannen der oberen Hautschicht im Bereich der Austrittsdüse führt zu einem zweistufigen Auseinanderziehen der verhornten Zellen der Hornschicht der Haut. Die erste Stufe besteht im Aufweiten der Haut in dem Augenblick, in dem das Ausblasrohr die Klebeschicht der Klebescheibe durchdringt. Die zweite Stufe besteht in einem partiellen Wegdrücken der Haut von der Klebescheibe. Das damit verbundene Eindellen der Haut bewirkt eine zusätzliche Dehnung der Haut. Auf diese Weise wird das Eindringen des Injektionsstrahls bei der Applikation erleichtert.

Weitere Einzelheiten der Erfindung ergeben sich aus den Unteransprüchen und den nachfolgenden Beschreibungen schematisch dargestellter Ausführungsbeispiele.
- Figur 1:: Zylinder-Kolben-Einheit mit angeformtem Ausblasrohr und Schutzgehäuse;
- Figur 2:: wie Figur 1, jedoch mit metallischem Ausblasrohr;
- Figur 3:: Schnitt durch den vorderen Bereich der Zylinder-Kolben-Einheit mit angeformtem Ausblasrohr nach der Abgabe der Injektionslösung;
- Figur 4:: Schnitt durch die mit der Klebescheibe nach Figur 1 und Figur 3 wieder verschlossene Haut;
- Figur 5:: wie Figur 3, jedoch mit metallischem Ausblasrohr;
- Figur 6:: Schnitt durch die mit der Klebescheibe nach Figur 2 und Figur 5 wieder verschlossene Haut;
- Figur 7:: Schnitt des metallischen Ausblasrohrs nach dem Aufsetzen auf die Patientenhaut vor dem vollständigen Durchstoßen der Klebescheibe;
- Figur 8:: wie Figur 7, jedoch nach dem vollständigen Durchstoßen der Klebescheibe und dem Spannen der Patientenhaut.

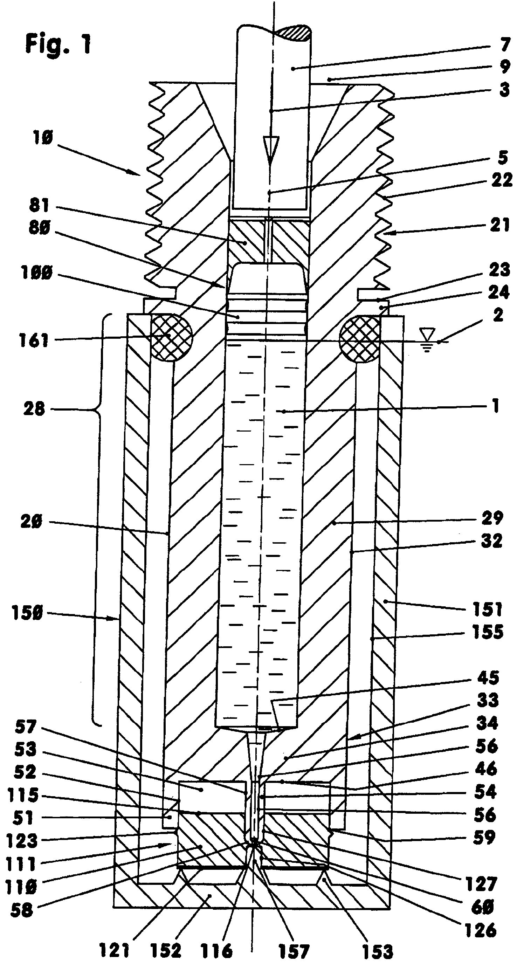

Die Figur 1 zeigt eine Zylinder-Kolben-Einheit (10) eines nadelfreien Injektors. Die Zylinder-Kolben-Einheit (10) besteht aus einem Zylinder (20) und einem z.B. zweiteiligen Kolben (80). Der Zylinder (20) ist beispielsweise zusätzlich von einem Schutzgehäuse (150) umgeben. Oberhalb des Kolbens (80) wird der untere Teil eines Kolbenbetätigungsstempels (7) gezeigt, der zu dem hier nicht dargestellten Injektor gehört. Der Zylinder (20) ist mittels seines im hinteren Bereich vorhandenen Außengewindes (22) oder mittels Schlitzen (23) am Injektor befestigt. Zwischen dem Zylinder (20) und dem Schutzgehäuse (150) ist zudem im Zylinderbodenbereich eine Klebescheibe (110) angeordnet.

Der z.B. einteilige Zylinder (20) besteht aus einem Gehäuseadapter (21), einem Rohrabschnitt (28) und einem Bodenabschnitt (33). Mit dem Gehäuseadapter (21) wird der Zylinder (20) in einem - nicht dargestellten - Injektorgehäuse fixiert. Hierzu weist seine radiale Außenwandung ein Außengewinde (22) und/oder mindestens zwei einander gegenüberliegende Schlitze (23) auf. Die Schlitze (23) haben z.B. eine Tiefe von 2 mm. Sie befinden sich am Gewindeende in unmittelbarer Nähe des Rohrabschnitts (28). Die Breite der Schlitze (23) beträgt z.B. 0,6 mm.

Zwischen den Schlitzen (23) und dem Rohrabschnitt (28) befindet sich ein Anschlagsteg (24), dessen Außendurchmesser z.B. identisch mit dem Gewindeaußendurchmesser sein kann. Der Außendurchmesser des Rohrabschnittes (28) ist mehr als doppelt so groß wie der Durchmesser der Innenwandung (31). Er ist so dimensioniert, dass sein Werkstoff mindestens einer Druckbelastung von 350 * 10⁵ Pa standhält.

An den Gehäuseadapter (21) schließt sich die Zylinderwandung (29) des Rohrabschnitts (28) an. Die Zylinderwandung (29) hat entlang der Rohrabschnittslänge z.B. eine konstante Wandstärke von 3,25 mm.

Der Bodenabschnitt (33) umfasst eine nach außen hin plane Bodenplatte (34), die der mittleren Wandstärke der Zylinderwandung (29) im Bereich des Rohrabschnitts (28) entspricht. Im äußeren Bereich der Bodenplatte (34) ist ein z.B. zylinderrohrförmiger Ringsteg (51) angeformt. Der Ringsteg (51), der einen Klebescheibenaufnahmeraum (53) umspannt, ist beispielsweise so hoch wie die Wandstärke der Bodenplatte (34). Die Wandstärke des Ringstegs (51) beträgt ca. ein Drittel der Wandstärke der Zylinderwandung (29) des Rohrabschnitts (28).

Im Zentrum der planen Bodenplatte (34) ist das die Austrittsdüse (60) tragende Ausblasrohr (54) angeordnet. Das Ausblasrohr (54), dessen Außendurchmesser z.B. 2,25 mm beträgt, hat ein vorderes Ende, das ca. einen Millimeter über den Ringsteg (51) übersteht. Zwischen der zumindest annähernd zylindrischen Außenwandung des Ausblasrohres (54) und der zylindrischen Innenwandung (52) des Ringstegs (51) liegt ein z.B. 3 mm tiefer Klebescheibenaufnahmeraum (53).

Die Stirnseite (58) des Ausblasrohres (54) ist zumindest bereichsweise sphärisch gekrümmt oder plan ausgeführt. Die vorn liegende Austrittsdüse (60) hat einen Innendurchmesser von 0,2 bis 0,4 mm, während der Innendurchmesser der zur Austrittsdüse (60) führenden Bohrung (56) zumindest in ihrer vorderen Hälfte ca. 0,5 bis 1 mm misst.

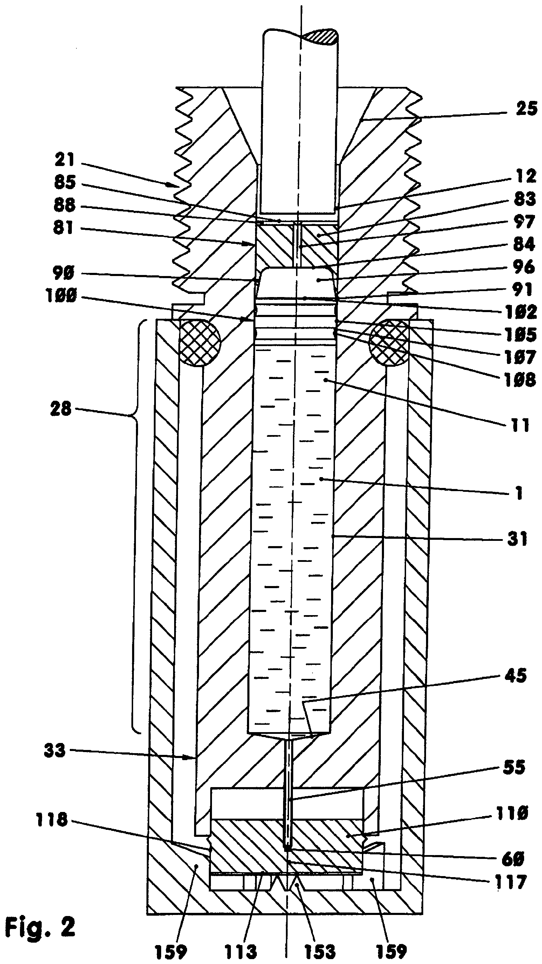

Gemäß Figur 2 ist die Zylinderinnenwandung (31) zumindest im Rohrabschnitt (28) zylindrisch gestaltet. Sie hat dort z.B. einen Innendurchmesser von 5,5 mm. Im Bereich des Gehäuseadapters (21) weitet sich die Zylinderinnenwandung (31) kegelstumpfmantelförmig auf. Der Kegelwinkel dieser Aufweitung (25) beträgt z.B. 50 Winkelgrade. Die Länge der Aufweitung (25) entspricht ca. einem Drittel der Länge des Gehäuseadapters (21).

Im Bereich des Bodenabschnitts (33) endet die Zylinderinnenwandung (31) in einem Zylinderboden (45), dessen Kegelwinkel z.B. 160 Winkelgrade beträgt. Nach Figur 1 befindet sich zwischen dem Zylinderboden (45) und der im Ausblasrohr (54) angeordneten Austrittsdüse (60) eine Ausblasbohrung (56), die zumindest im Bereich vor der Austrittsdüse (60) eine zylindrische Wandung hat. Der hintere, dem Zylinderboden (45) zugewandte Abschnitt der Bohrung (56) ist im Ausführungsbeispiel als Kegelstumpf ausgebildet. Er hat einen Kegelwinkel von z.B. 12 Winkelgraden. Er erstreckt sich beispielsweise im Bereich der Wandung der Bodenplatte (34).

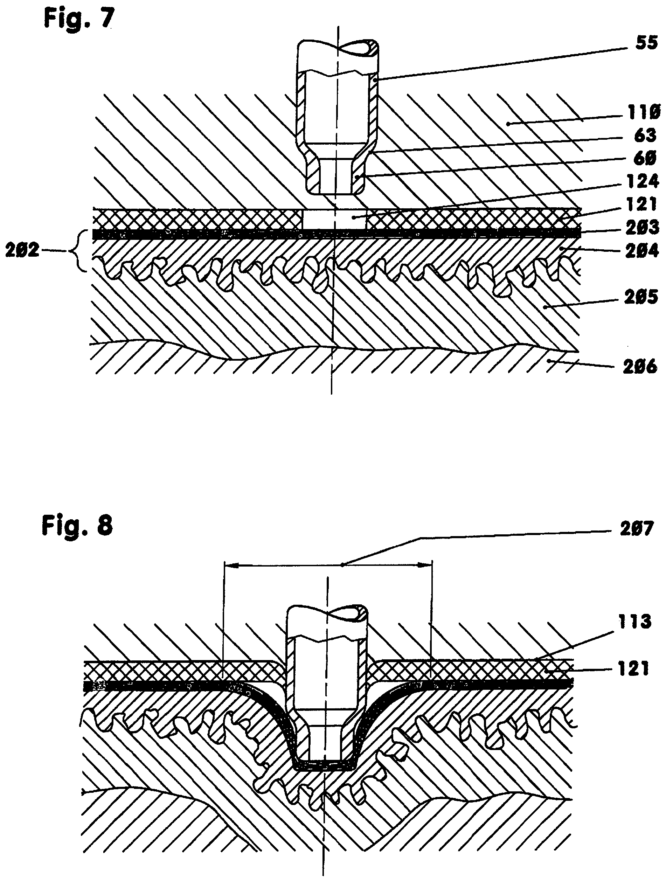

Anstelle des angeformten Ausblasrohres (54) kann auch ein dünnwandiges Rohr (55) verwendet werden, vgl. Figur 2, an dessen vorderem Ende eine Austrittsdüse (60) angeordnet ist. Das dünnwandige Rohr (55), das z.B. aus einem nichtrostenden Stahl herstellt wird, hat z.B. einen Außendurchmesser von 0,5 mm. Die Wandstärke des Ausblasrohres (55) beträgt hinter der Austrittsdüse (60) z.B. 0,05 mm. Der Düsen- bzw. der Innendurchmesser des Ausblasrohrs (55) entspricht in der Regel den aus der vorherigen Variante bekannten Werten. Nach den Figuren 7 und 8 misst der Innendurchmesser z.B. 0,2 mm, während der Außendurchmesser 0,36 mm beträgt. Um diese Austrittsdüse (60) herzustellen, wird der vordere Bereich des Ausblasrohres (55) auf einer Länge von ca. 0,325 mm durch Materialstauchen reduziert, so dass unmittelbar hinter der Austrittsdüse (60) eine Verjüngung (63) entsteht.

Das freie Düsenende ist z.B. mit einem Radius von 0,05 mm abgerundet, um bei der Anwendung die Haut (200) nicht zu schädigen.

Zwischen dem Ausblasrohr (55) und dem Ringsteg (51) ist nach Figur 1 im vorderen Bereich des Klebescheibenaufnahmeraums (53) eine Klebescheibe (110) angeordnet. Sie hat eine Materialstärke, die mindestens 0,5 mm größer ist als die Tiefe des Klebescheibenaufnahmeraums (53). Die Klebescheibe (110) hat zwei gegenläufige zentrale Sacklochbohrungen (126, 127). Beide Sacklochbohrungen (126, 127) enden im mittleren Bereich der Klebescheibe (110) vor einer als Dichtelement dienenden, nach vorn gewölbten, dünnwandigen Membrane (116). Die hintere Sacklochbohrung (127) umgibt den vorderen Bereich des Ausblasrohres (55) dicht und eng anliegend. Die Membrane (116) sitzt wie eine Kappe eng anschmiegend auf der ggf. sphärisch gewölbten vorderen Stirnfläche (58) des Ausblasrohres (55). Ihre Wandstärke beträgt z.B. 0,13 mm. Die vordere Sacklochbohrung (126) hat nach Figur 1 aufgrund der fehlenden Aufdehnung durch das Ausblasrohr (54) einen Durchmesser, der ca. 0,5 -1 mm kleiner ist als der Durchmesser der hinteren Sacklochbohrung (127).

Für die Zylindervariante mit dem eingeklebten oder umspritzten Ausblasrohr (55) wird eine Klebescheibe (110) verwendet, die keine Bohrung aufweist. Der vor dem Ausblasrohr (55) gelegene Bereich der Klebescheibe (110) ersetzt als Dichtbereich (117) die Membrane (116).

Die im Wesentlichen zylindrische Außenwandung der Klebescheibe (110) ist an der zylindrischen Innenwandung (52) des Ringstegs (51) geführt. Nach Figur 1 hat die Klebescheibe (110) im oberen Bereich ihrer Außenwandung einen radial, z.B. 0,5 mm, überstehenden Umlaufsteg (123), über den sie an der vorderen Innenkante (59) des Ringstegs (51) elastisch anliegt.

Zur Positionierung der Klebescheibe (110) am Ringsteg (51) des Bodenabschnitts (33), kann letzterer auch einen im vorderen Bereich des Ringstegs (51) angeformten, radial nach innen ragenden Steg aufweisen, der in eine entsprechende Ringnut der Klebescheibe (110) elastisch hineinragt.

Die z.B. aus Gummi oder einem anderen Elastomer gefertigte Klebescheibe (110) ist an ihrer plan ausgeführten vorderen Stirnseite mit einer z.B. aus einem Haftkleber bestehenden Klebeschicht (121) ausgestattet. Die restlichen Oberflächenbereiche haben eine gute Gleitfähigkeit, da die Klebescheibe (110) zumindest partiell mit Silikonöl behandelt oder teflonisiert wird. Bei der für das Ausblasrohr (54) geeigneten Variante, vgl. Figuren 1, 3 und 4, ist die Klebeschicht (121) im Bereich der Bohrung (126) ausgespart. Bei der Variante für das metallische Ausblasrohr (55) kann die Klebeschicht (121) eine zentrale Ausnehmung (124) haben, deren Durchmesser zumindest größer ist, als der Außendurchmesser des Ausblasrohres (55) im Bereich der Austrittsdüse (60), vgl. Figur 7.

Der Haftkleber der Klebescheibe (121) ist so ausgelegt, dass seine Klebekraft gegenüber der Klebescheibe (110) um mindestens 50% größer ist als gegenüber einer desinfizierten Hautoberfläche (201).

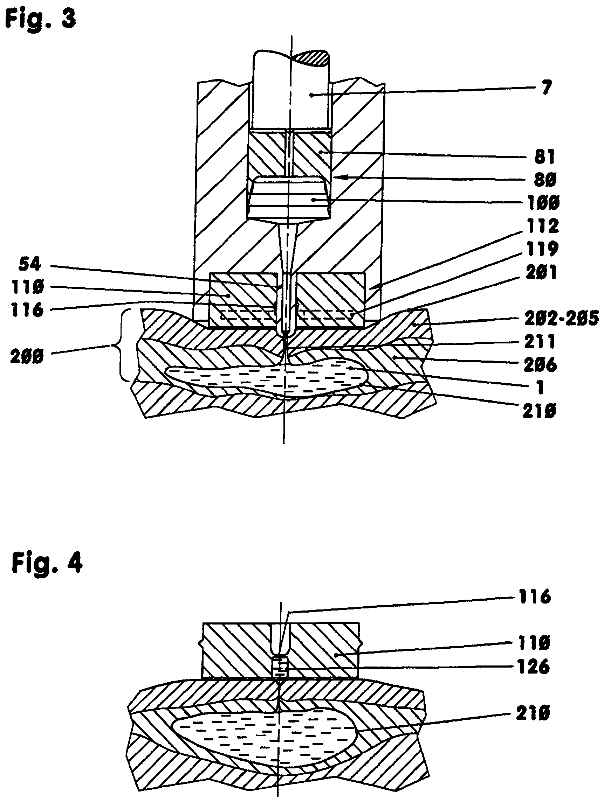

In die Klebescheibe (110) kann eine Versteifungsscheibe (119) eingesetzt werden. Sie ist in Figur 3 gestrichelt dargestellt. Diese z.B. umspritzte oder einvulkanisierte Versteifungsscheibe (119) hat eine Wandstärke von z.B. 0,5 -1 mm. Sie ist beispielsweise aus einem üblichen Eisen- oder Buntmetall gefertigt. Ihre Bohrung ist mindestens 1 mm größer als der Außendurchmesser des Ausblasrohres (54). Der Außendurchmesser der Versteifungsscheibe (119) ist z.B. 1 - 2 mm kleiner als der Außendurchmesser der Klebescheibe (110). Die hier in die Klebescheibe (110) integrierte Versteifungsscheibe (119) ist z.B. 0,5 bis 1 mm hinter der vorderen Klebeschicht 121) positioniert. Die Mittellinien der Klebescheibe (110) und der Versteifungsscheibe (119) sind deckungsgleich.

Ggf. hat die Klebescheibe (110) seitlich mindestens eine parallel zur Mittellinie (5) orientierte Kerbe, die es beim Einschieben der Klebescheibe (110) in den Klebescheibenaufnahmeraum (53) ermöglicht, die dort vorhandene Luft problemlos zu verdrängen. Die Luft kann auch über eine im Ringsteg (51), in der Nähe der Stirnfläche (46) des Bodenabschnitts (33), angeordnete Bohrung entweichen.

Ein topfförmiges Schutzgehäuse (150), ein Sterilverschluss, umgibt nach den Figuren 1 und 2 den Rohrabschnitt (28) und den Bodenabschnitt (33) mit der eingesetzten Klebescheibe (110) des Zylinders (20). Es besteht hier aus einem rohrförmigen Mantel (151) und einem planen Boden (152). In den gezeigten Ausführungsbeispielen ist das Schutzgehäuse (150) aufgrund seiner Formgebung aus dem Kunststoff Cycloolefin Copolymer (COC) gefertigt. Dieser Werkstoff hat eine besonders niedrige Gas- und Wasserdampfdurchlässigkeit. Bei einer einfacheren Formgebung kann das Schutzgehäuse (150) aus Glas gefertigt werden.

Im Bereich des Rohrabschnitts (28) beträgt der Abstand zwischen dessen Außenwandung (32) des Rohrabschnitts (28) und der Innenwandung (155) des Schutzgehäuses (150) z.B. 1,5 mm. Der axiale Abstand zwischen dem Boden (152) des Schutzgehäuses (150) und der Klebescheibe (110) beträgt nach Figur 1 z.B. 1 mm.

Am Zylinder (20) ist das Schutzgehäuse (150) an zwei Stellen lösbar fixiert. Die erste Stelle liegt am Übergang zwischen dem Rohrabschnitt (28) und dem Anschlagsteg (24) des Zylinders (20). Dort befindet sich, nach Figur 1, ein in einer Kerbe des Zylinders (20) sitzender O-Ring (161), über den das Schutzgehäuse (150) gegenüber dem Zylinder (20) abgedichtet ist. Zugleich zentriert der O-Ring (161) das Schutzgehäuse (150) am Zylinder (20). Anstelle eines konventionellen O-Rings (161) kann auch ein Quadring, ein Profilring oder dergleichen verwendet werden.

Der Dichtring (161) wird bei der Montage zwischen dem Schutzgehäuse (150) und dem Zylinder (20) verklemmt, so dass er neben der Dichtfunktion auch problemlos eine Zentrier- und Haltefunktion übernehmen kann. Ggf. kann der Dichtring (161) auch durch einen abdichtenden, zähbleibenden Klebstoff ersetzt werden.

Die zweite Stelle zur Abstützung des Schutzgehäuses (150) am Zylinder (20) befindet sich mittig im Boden (152) des Schutzgehäuses (150). Dort ist ein zentraler Stützzapfen (157) angeordnet, der so weit in die Sacklochbohrung (126) des Bodenabschnitts (33) hineinragt, dass er die dortige Membrane (116) kontaktiert. Der Stützzapfen (157) fixiert in radialer Richtung das vordere Ende des Schutzgehäuses (150) über die Klebescheibe (110), die sich am Ringsteg (51) des Zylinders (20) abstützt.

Zur axialen Stützung der Klebescheibe (110) ist zudem am Boden (152) ein ringförmiger Stützsteg (153) angeordnet, der mit seiner oberen, kreisförmigen Kante an der Klebescheibe (110), in deren Randbereich, anliegt. Die Kante ist hierbei so schmal, dass sie gegenüber der Klebescheibe (110) nur eine geringe Haftkraft entwickelt.

Um bei der Variante mit dem stählernen Ausblasrohr (55), vgl. Figur 2, das Schutzgehäuse (150) im vorderen Bereich am Zylinder (20) auch in Radialrichtung abstützen zu können, weist das Schutzgehäuse (150) z.B. fünf radial orientierte Stützrippen (159) auf. Diese am Umfang des Mantels (151) äquidistant verteilten Stützrippen (159) sind beispielsweise am Boden (152) und am Mantel (151) angeformt. Die Stützrippen (159) haben radiale Innenflächen, mit denen sie an der zylindrischen Außenfläche (118) der Klebescheibe (110) anliegen.

Nach den Figuren 1 und 2 ist der Zylinder (20) mit einer Injektionslösung (1) teilbefüllt. Der Flüssigkeitsspiegel (2) der Injektionslösung (1) befindet sich im Übergangsbereich zwischen dem Gehäuseadapter (21) und dem Rohrabschnitt (28). Auf dem Flüssigkeitsspiegel (2) ist blasenfrei und steril ein scheibenförmiger Dichtkörper (100) aufgesetzt, der unter einer radialen Klemmwirkung dichtend an der Zylinderinnenwandung (31) anliegt. Hinter dem Dichtkörper (100) ist ein topfförmiger Treibkörper (81) angeordnet. Der Treibkörper (81) liegt dabei am Dichtkörper (100) partiell an oder er hat einen Abstand von z.B. 0,2 bis 0,5 mm.

Der Dichtkörper (100) ist hier eine Scheibe, deren unverformter Durchmesser z.B. doppelt so groß ist wie seine Scheibendicke. Am Umfang kann die Scheibe (100) ein Rillenprofil (107) aufweisen, das z.B. zwei Rillen (108) hat, vgl. Figur 2. Das Rillenprofil (107) ist hier beispielsweise so gestaltet, dass der Dichtkörper (100) im Querschnitt beidseitig als Schnittprofil eine Wellenlinie mit zwei, die Rillen (108) formenden Wellentälern aufweist. Die Wellenlinie ist hierbei aus Kreisbögen zusammengesetzt.

Da der Dichtkörper (100) ein Elastomerkörper ist, sind die Wellenberge der gesetzten Dichtscheibe abgeplattet, vgl. Figuren 1, 2, 4 und 6.

Der topfförmige Treibkörper (81), dessen Länge z.B. seinem Außendurchmesser entspricht, besteht aus einer scheibenförmigen Schlagplatte (83) und einer angeformten Schürze (90). Die Dicke der Schlagplatte (83) ist hierbei geringfügig größer als die Länge der Schürze (90), vgl. Figur 2.

Die Schlagplatte (83), auf die beim Auslösen des Injektors der Kolbenbetätigungsstempel (7) aufschlägt, hat mindestens eine z.B. zentrale Bohrung (97), die die vor und hinter dem Treibkörper (81) gelegenen Zylinderraumbereiche (11, 12) miteinander mit minimaler Drosselwirkung verbindet. Die Bohrung (97), deren minimaler Durchmesser zwischen 1 und 2 mm liegt, endet nach den Ausführungsbeispielen an der hinteren Stirnseite (85) des Treibkörpers (81), z.B. in einem Kanalkreuz (88) aus zwei sich im Bereich der Bohrung (97) schneidenden Kanälen. Die Kanäle des Kanalkreuzes (88) haben z.B. jeweils einen halbkreisförmigen Querschnitt, wobei der Durchmesser der Querschnitte z.B. dem Durchmesser der Bohrung entspricht.

An der vorderen Stirnseite (84) der Schlagplatte (83) schließt sich die als elastische Dichtlippe ausgebildete Schürze (90) an. Die Wandung der Schürze (90) verjüngt sich, ausgehend von der Stirnseite (84), hin zur vorderen, äußeren Dichtkante (91), die elastisch an der Zylinderinnenwandung (31) in jedem Betriebszustand des Injektors anliegt. Im eingebauten Zustand umschließen die Schürze (90) und die vordere Stirnseite (84) einen Eintauchhohlraum (96). Letzterer hat im Wesentlichen die Form eines Kegelstumpfes, dessen Kegelwinkel z.B. 20 Winkelgrade misst.

Der Kolben (80), also die Kombination aus dem Treibkörper (81) und dem Dichtkörper (100), ermöglicht ein einfaches blasenfreies, steriles Befüllen und Verschließen der Zylinder-Kolben-Einheit (10) in Verbindung mit einem Ausstoßvorgang beim Auslösen des Injektors, der einem sehr hohen Verdichtungsstoß von bis zu 350 * 10⁵ Pa standhält.

Wird der Injektor für die Injektion vorbereitet, wird das Schutzgehäuse (150) nach vorn vom Zylinder (20), z.B. mittels Handkraft, abgezogen. Hierbei bleibt die Klebescheibe (110) im Bodenabschnitt (33) des Zylinders (20) haften. Auch der Dichtring (161) bleibt an der Außenwandung (32) des Zylinders (20) zurück.

Um die Applikation der Injektionslösung durchführen zu können, wird der Injektor mit der Klebescheibe (110) voraus auf die Hautoberfläche (201) des Patienten aufgesetzt. Dabei verklebt die Klebescheibe (110), die sich noch in ihrer Einbauposition (111) befindet, mit ihrer Klebeschicht (121), die z.B. bei einem Durchmesser von 10 mm eine Fläche von ca. 75 mm² hat, mit der Hautoberfläche (201). Nach dem Ausführungsbeispiel, vgl. Figur 1, ist nur das Zentrum der Klebescheibe (110) nicht mit einer Klebeschicht (121) ausgestattet. Die klebeschichtfreie Stelle hat z.B. einen Durchmesser von 1 bis 2 mm.

Durch die Anpresskraft des Injektors wird die Klebescheibe (110) so belastet, dass sie - unter einem Überwinden der Sperrwirkung des Umlaufstegs (123) und unter einem Aufreißen der Membrane (116) - entlang des Ausblasrohrs (54) in Richtung des Bodenabschnitts (33) verschiebt. Während sich hierbei die vordere Stirnfläche (58) des Ausblasrohres (54) auf die Haut zubewegt, dehnt das Ausblasrohr (54) die vordere Sacklochbohrung um ca. 0,5 -1 mm auf. Diese Aufdehnung führt dazu, dass die mit der Klebeschicht (121) verklebte Hautschicht in einer ersten Stufe - allein durch das Aufweiten der Sacklochbohrung (126) - aktiv vorgespannt wird.

Bei der weiteren Vorwärtsbewegung des Injektors rutscht die Klebescheibe (110) weiter in den Klebescheibenaufnahmeraum (53) hinein, um sich mit ihrer hinteren Stirnseite (115) an der Stirnfläche (46) des Bodenabschnitts (33) anzulegen. Die Klebescheibe (110) befindet sich jetzt in ihrer Applikationsposition (112). Sie füllt nun den Klebescheibenaufnahmeraum (53) komplett aus.

Durch diesen weiteren Hub der Klebescheibe (110) wird zum einen durch die Erhöhung des Anpressdrucks die Verklebung zwischen der Klebeschicht (121) und der Haut des Patienten verstärkt und zum anderen tritt das Ausblasrohr (54) einige zehntel Millimeter aus der Klebescheibe (110) hervor, vgl. Figur 3.

Dabei drückt die vordere Stirnseite (58) des hervorstehenden Ausblasrohres (54) mit der beispielsweise nahezu sphärisch gekrümmten Stirnfläche (58) eine Vertiefung in die Haut (200), wodurch sich die zweite Stufe der Hautdehnung einstellt. Die hierbei entstehende Beulentiefe, das ist der Abstand zwischen der unteren Klebeschicht (121) und dem vordersten Punkt oder der vordersten Kante des Ausblasrohres (54), entspricht z.B. dem halben Außendurchmesser des Ausblasrohres (54). In diesem Fall wird die Hautoberfläche (201) im Bereich der Stirnfläche (58) des Ausblasrohres (54) in Summe aktiv um ca. 100% gedehnt.

Wird der Injektor aus Figur 2 benutzt, so ergibt sich prinzipiell eine vergleichbare zweistufige Hautdehnung, wie bei dem Beispiel nach Figur 1. Aufgrund des relativ schlanken Ausblasrohres (55) mit seiner Verjüngung (63) im Bereich der Austrittsdüse (60) gibt es je nach Hauttyp zwei verschiedene Anlagebedingungen für die zu dehnende Haut (200).

Bei einer ersten Anlagebedingung schmiegt sich die Haut (200) beim Heraustreten des Ausblasrohres (55) an der Außenwandung (57) an, so dass die Oberfläche (201) der gedehnten Haut (200) die Form einer gestuften Büchse annimmt, wobei die Büchsenstufung durch die Verjüngung (63) des Ausblasrohres (55) zustande kommt. Bei der zweistufigen Hautdehnung ergibt sich bei dieser Variante z.B. ein Dehnungsfaktor von 3,5 bis 4.

Bei einer zweiten Anlagebedingung wird die Hornschicht (203) - ohne die Haut (200) zu schädigen - durch die Austrittsdüse (60) bereichsweise von der Klebeschicht (121) abgehoben bzw. gelöst. Der Abhebebereich hat z. B. einen Durchmesser (207), der bis zu 1 mm betragen kann.

Die Haut (200) wird hier unter der Entstehung einer größeren Dehnung im Bereich um die Austrittsdüse (6) eine z.B. trichterähnliche Gestalt annehmen, vgl. Figur 8. Hierbei werden die Epidermis (202) und die Dermis (205) bis in die Unterhaut (206) ausgebeult. Bei den geometrischen Verhältnissen nach Figur 8, bei der die Hornschichtbeulung ca. dem halben Durchmesser (207) der von der Klebeschicht (121) abgelösten Hautoberfläche (201) entspricht, stellt sich dort im Bereich der trichterähnlichen Beulung eine Hornschichtdehnung von 60 bis 70% ein.

Gleichzeitig wird der Injektor, durch das Aufdrücken auf die Haut (200), ausgelöst. Der mittels einer mechanischen oder pneumatischen Feder vorgespannte Kolbenbetätigungsstempel (7) belastet schlagartig den Kolben (80), um mit der Injektionslösung (1) per Hochgeschwindigkeitsstrahl die gespannten Haut des Patienten zu durchschlagen.

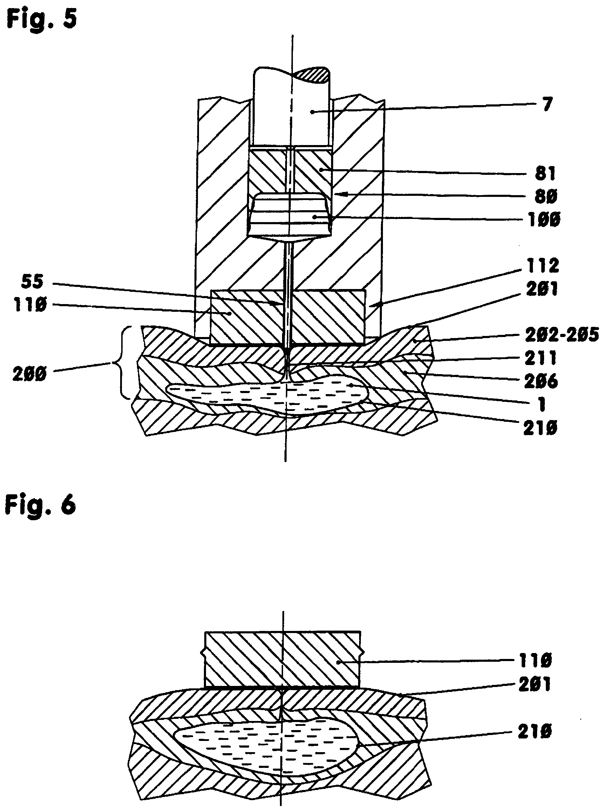

Dabei schlägt der Kolbenbetätigungsstempel (7) zunächst mit großer Wucht gegen den Treibkörper (81), siehe Pfeilrichtung (3) in Figur 1. Der Treibkörper (81) wird gegen die nahezu inkompressibel auf dem Flüssigkeitsspiegel (2) aufliegende Dichtscheibe (100) gepresst. Hierbei schiebt sich die Schürze (90) entlang der Zylinderinnenwandung (31) über die profilierte Außenwandung (105) der Dichtscheibe (100). Die Dichtscheibe (100) taucht in den Eintauchhohlraum (96) des Treibkörpers (81) ein, vgl. Figur 3 und 5. Die dabei verdrängte Luft strömt über die Bohrung (97) und das Kanalkreuz (88) am Kolbenbetätigungsstempel (7) entlang in die äußere Injektorumgebung (9).

Nun bilden die Dichtscheibe (100) und der Treibkörper (81) einen quasistarren Verbund, den Kolben (80), der die Injektionslösung (1) vor sich herschiebt. Die Abdichtung gegenüber der Zylinderinnenwandung (31) übernimmt die Dichtkante (91) der Schürze (90), die durch die flüssigkeitsdruckbelastete Dichtscheibe (100) verstärkt angepresst wird. Da der Gleitreibungskoeffizient der Dichtkante (91), aufgrund des verwendeten Treibkörperwerkstoffes, kleiner ist als der Gleitreibungskoeffizient der Dichtscheibe (100), ergibt sich trotz hoher Dichtwirkung ein geringer Gleitreibungswiderstand.

Durch den hohen Flüssigkeitsdruck, er misst z.B. für die subkutane Injektion bei einer Ausströmgeschwindigkeit von ca. 150 m/sec mindestens 250 * 10⁵ Pa, durchdringt der Flüssigkeitsstrahl die Epidermis (202) und die Dermis (205), die zusammen z.B. bis zu 4 mm dick sind, vgl. Figuren 3 und 5. Ein durch den Flüssigkeitsstrahl erzeugter Einströmkanal (211) endet bei der subkutanen Injektion erst in der Unterhaut (206). In dem von Kapillargefäßen durchzogenen fettgewebsreichen Bindegewebe der Subcutis (206) entsteht dann eine vom Flüssigkeitsstrahl gespeiste, z.B. diskusförmige, Injektionslösungsansammlung (210).

Diese unter Druck stehende Injektionslösungsansammlung (210) will sich über den Einströmkanal (211) sofort zumindest teilweise entleeren, wenn der Injektor mit allen seinen Teilen von der Haut (200) abgehoben wird. Dieser störenden Entteerung widersetzt sich die Haut nach der Wegnahme des Injektors schon dadurch, dass sich die Hautdehnung wieder abbaut und dabei den Einströmkanal (211), zumindest bei kleineren Injektionsmengen, großteils verschließt.

Im vorliegenden Fall bleibt nach der Wegnahme des Injektors zusätzlich die Klebescheibe (110) aufgrund ihrer Klebeschicht (121) zurück, bis sie später separat entfernt wird. Bis es jedoch zeitlich so weit ist, verschließt ihre zuvor eingerissene Membrane (116) die Sacklochbohrung (126) der Klebescheibe (110) aufgrund ihrer entgegen einer möglichen Ausströmrichtung orientieren Wölbung. Eine kleine Teilmenge der Injektionslösung staut sich ggf. als Verlust in der vorderen Sacklochbohrung (126) vor der Membrane (116). Der Patient entfernt die Klebescheibe (110), einschließlich der daran haftenden Klebeschicht (121), sobald sich die injektionsbedingte Wölbung der Haut weitgehend zurückgebildet hat.

Die Variante nach Figur 2 zeigt das gleiche Verhalten beim Einbringen der Injektionslösung, vgl. Figur 5. Allerdings ist das externe Verschließen des Einströmkanals (211) nach Figur 6 effektiver als bei der Lösung nach Figur 4. Da die Klebescheibe (110) vor der Injektion durch das dünne stählerne Ausblasrohr (55) nur unter Erzeugung eines schmalen Kanales aufgestochen wird, verschließt sich dieser nach der Wegnahme des Injektors sofort vollständig. Auch hier wird die Klebescheibe (110) erst von der Hautoberfläche (201) abgezogen, wenn sich die Injektionslösung (1) in dem die Injektionslösungsansammlung (210) umgebenden Gewebe verteilt hat.

### Bezugszeichenliste:

- 1: Injektionslösung
- 2: Flüssigkeitsspiegel
- 3: Pfeilrichtung bei Injektorauslösung
- 5: Mittellinie
- 7: Kolbenbetätigungsstempel
- 9: Umgebung

- 10: Zylinder-Kolben-Einheit
- 11: Zylinderraumbereich, vor dem Kolben
- 12: Zylinderraumbereich, hinter dem Kolben

- 20: Zylinder
- 21: Gehäuseadapter
- 22: Außengewinde
- 23: Schlitze
- 24: Anschlagsteg
- 25: Aufweitung, innen

- 28: Rohrabschnitt
- 29: Zylinderwandung
- 31: Innenwandung, radial
- 32: Außenwandung, radial
- 33: Bodenabschnitt
- 34: Bodenplatte

- 45: Zylinderboden, Innenseite des Zylinderbodens
- 46: Stirnfläche des Bodenabschnitts, vorn
- 51: Ringsteg
- 52: Innenwandung, zylindrisch
- 53: Klebescheibenaufnahmeraum
- 54: Ausblasrohr, Kunststoff
- 55: Ausblasrohr, Rohr, Stahlrohr
- 56: Bohrung, Innenbohrung
- 57: Außenwandung
- 58: Stirnseite, Stirnfläche
- 59: Kante, vordere
- 60: Düse, Austrittsdüse
- 63: Verjüngung

- 80: Kolben, Kombination aus (81) und (100)
- 81: Treibkörper
- 83: Schlagplatte
- 84: Stirnseite, vorn
- 85: Stirnseite, hinten
- 88: Kanalkreuz

- 90: Schürze, elastisch; Dichtlippe
- 91: Kante, Dichtkante
- 96: Eintauchhohlraum, Hohlraum
- 97: Ausnehmung, Bohrung, zentral

- 100: Dichtkörper, Dichtscheibe
- 102: Stirnseite, hinten
- 105: Außenwandung, profiliert
- 107: Rillenprofil
- 108: Rille
- 110: Klebescheibe, Elastomerscheibe
- 111: Einbauposition
- 112: Applikationsposition
- 113: Stirnseite, vorn; Stirnfläche
- 115: Stirnseite, hinten
- 116: Dichtelement, Membrane
- 117: Dichtbereich
- 118: Außenfläche, radial
- 119: Versteifungsscheibe
- 121: Klebeschicht, vorn, Haftkleber, Klebebeschichtung
- 123: Umlaufsteg
- 124: Ausnehmung in (121)
- 126: Bohrungsabschnitt, vorn
- 127: Bohrungsabschnitt, hinten
- 150: Schutzgehäuse, Glas; Schale, außen; Sterilverschluss
- 151: Mantel, rohrförmig
- 152: Boden, plan
- 153: Stützsteg
- 155: Innenwandung
- 157: Stützzapfen
- 159: Stützrippen
- 161: O-Ring

- 200: Haut
- 201: Hautoberfläche
- 202: Epidermis

- 121: Klebeschicht, vom, Haftkleber, Klebebeschichtung
- 123: Umlaufsteg
- 124: Ausnehmung in (121)
- 126: Bohrungsabschnitt, vorn
- 127: Bohrungsabschnitt, hinten
- 203: Hornschicht (Stratum Corneum)
- 204: Hornbildungs- und Regenerationsschicht
- 205: Papillar- und Geflechtsschicht (Dermis)
- 206: Unterhaut (Subcutis)
- 207: Durchmesser der abgelösten Hautoberfläche (201)

- 210: Injektionslösungsansammlung
- 211: Einströmkanal

## Patentansprüche

1. Zylinder-Kolben-Einheit (10), mit mindestens einem, eine Injektionslösung aufnehmenden Zylinder (20), mindestens einem Kolben (80) und einer im Bereich der freien Stirnseite des Zylinders (20) angeordneten Klebebeschichtung (121), wobei
i.) der Zylinder (20) einen Bodenabschnitt (33) aufweist, an dem ein Ausblasrohr (54, 55) angeordnet ist,
ii.) am Ausblasrohr (54, 55) und/oder am Bodenabschnitt (33) eine in Richtung der Mittellinie (5) der Zylinder-Kolben-Einheit (10) zwischen einer Einbauposition (111) und einer Applikationsposition (112) verschiebbare, elastische Klebescheibe (110) angeordnet ist,
iii.) die Klebescheibe (110) auf ihrer dem Bodenabschnitt (33) abgewandten Stirnseite (113) eine Klebebeschichtung (121) zur Verklebung mit der Haut (200) aufweist,
iv.) die Klebescheibe (110) in der Einbauposition (111) mindestens ein Dichtelement (116) oder einen Dichtbereich (117) zum Verschließen des Ausblasrohres (54, 55) aufweist, dessen Wirkung in der Applikationsposition nicht mehr vorhanden ist,
v.) in der Applikationsposition (112) die vordere Kante (59) der freien Stirnseite (58) des Ausblasrohres (54, 55) zur Eindellung der Haut (200) mindestens 0,5 mm über die Klebebeschichtung (121) der Klebescheibe (110) übersteht,
vi.) durch das Aufdehnen der Klebescheibe (110) und das Überstehen des Ausblasrohres (54, 55) die Haut (200) spannbar ist und
vii.) die Klebescheibe (110) nach Wegnahme der Zylinder-Kolben-Einheit auf der Haut (200) zurückbleibt und separat entfernbar ist.

2. Zylinder-Kolben-Einheit gemäß Anspruch 1,
**dadurch gekennzeichnet,**
**dass** die sich in Einbauposition (111) befindende Klebescheibe (110) mindestens einen Millimeter von der ihr zugewandten vorderen Stirnseite (46) des Bodenabschnitts (33) entfernt ist.

3. Zylinder-Kolben-Einheit gemäß Anspruch 1,
**dadurch gekennzeichnet,**
**dass** die Klebescheibe (110) in der Applikationsposition (112) an der vorderen Stirnseite (46) des Bodenabschnitts (33) anliegt.

4. Zylinder-Kolben-Einheit gemäß Anspruch 1,
**dadurch gekennzeichnet,**
**dass** die Außenwandung (57) des Ausblasrohres (54, 55) zumindest bereichsweise zylindrisch geformt ist.

5. Zylinder-Kolben-Einheit gemäß Anspruch 1,
**dadurch gekennzeichnet,**
**dass** der Bodenabschnitt (33) einen Ringsteg (51) aufweist, der sich nach vorn in einer Verlängerung der Zylinderaußenwandung (32) erstreckt.

6. Zylinder-Kolben-Einheit gemäß Anspruch 5,
**dadurch gekennzeichnet,**
**dass** der Ringsteg (51) eine zylindrische Innenwandung (52) aufweist.

7. Zylinder-Kolben-Einheit gemäß Anspruch 1,
**dadurch gekennzeichnet,**
**dass** die Klebescheibe (110) zwei koaxial hintereinander liegende Bohrungen (126) und (127) aufweist, die durch ein Dichtelement (116) in Form einer Membran getrennt sind.

8. Zylinder-Kolben-Einheit gemäß Anspruch 1,
**dadurch gekennzeichnet,**
**dass** die Klebescheibe (110) aus Gummi oder einem Elastomer besteht oder partiell mit Silikon behandelt oder teflonisiert ist.

9. Zylinder-Kolben-Einheit gemäß Anspruch 5,
**dadurch gekennzeichnet,**
**dass** die Klebescheibe (110) einen radial überstehenden Umlaufsteg (123) aufweist, welcher an der vorderen Innenkante (59) des Ringstegs (51) anliegt.

## Claims

1. A cylinder-piston unit (10), having at least one cylinder (20) receiving an injection solution, at least one piston (80), and an adhesive coating (121) arranged in the region of the free end face of the cylinder (20), wherein
i.) the cylinder (20) has a base portion (33), on which an exhaust pipe (54, 55) is arranged,
ii.) a resilient adhesive disc (110) displaceable in the direction of the centre line (5) of the cylinder-piston unit (10) between an installation position (111) and an application position (112) is arranged on the exhaust pipe (54, 55),
iii.) the adhesive disc (110) has, on its end face (113) facing away from the base portion (33), an adhesive coating (121) for adhering to the skin (200),
iv.) the adhesive disc (110) in the installation position (111) has at least one sealing element (116) or a sealing region (117) for closing the exhaust pipe (54, 55),
the effect of which is no longer provided in the application position,
v.) in the application position (112) the front edge (59) of the free end face (58) of the exhaust pipe (54, 55) protrudes at least 0.5 mm beyond the adhesive coating (121) of the adhesive disc (110) in order to dent the skin (200),
vi.) the skin (200) can be stretched taut by the expansion of the adhesive disc (110) and the protrusion of the exhaust pipe (54, 55), and
vii.) the adhesive disc (110) remains on the skin (200) after the cylinder-piston unit has been taken away and can be removed separately.

2. The cylinder-piston unit according to claim 1,
**characterised in that**
the adhesive disc (110) disposed in the installation position (111) is distanced by at least one millimetre from the front end face (46) of the base portion (33) facing towards said adhesive disc.

3. The cylinder-piston unit according to claim 1,
**characterised in that**
the adhesive disc (110) bears against the front end face (46) of the base portion (33) in the application position (112).

4. The cylinder-piston unit according to claim 1,
**characterised in that**
the outer wall (57) of the exhaust pipe (54, 55) is at least partly cylindrical.

5. The cylinder-piston unit according to claim 1, **characterised in that**
the base portion (33) has an annular rib (51), which extends forwards in an extension of the cylinder outer wall (32).

6. The cylinder-piston unit according to claim 5,
**characterised in that**
the annular rib (51) has a cylindrical inner wall (52).

7. The cylinder-piston unit according to claim 1,
**characterised in that**
the adhesive disc (110) has two bores (126) and (127) arranged coaxially one behind the other, which are separated by a sealing element (116) in the form of a membrane.

8. The cylinder-piston unit according to claim 1,
**characterised in that**
the adhesive disc (110) consists of rubber or an elastomer or is treated in part with silicone or is Teflon-coated.

9. The cylinder-piston unit according to claim 5,
**characterised in that**
the adhesive disc (110) has a radially protruding peripheral rib (123), which bears against the front inner edge (59) of the annular rib (51).

## Revendications

1. Unité cylindre-piston (10) avec au moins un cylindre (20) réceptionnant une solution d'injection, au moins un piston (80) et un revêtement adhésif (121) disposé dans la région de la partie frontale libre du cylindre (20),
où
i.) le cylindre (20) présente une partie de fond (33), sur laquelle est disposé un tuyau d'évacuation par soufflage (54, 55),
ii.) un disque adhésif (110) élastique est disposé sur le tuyau d'évacuation par soufflage (54, 55) et/ou sur la partie de fond (33), pouvant coulisser en direction de la ligne médiane (5) de l'unité cylindre-piston (10) entre une position de montage (111) et une position d'application (112),
iii.) le disque adhésif (110) présente un revêtement adhésif (121) sur sa partie de fond (33) opposée à la partie frontale (113) pour le collage avec la peau (200),
iv.) le disque adhésif (110) présente au moins un joint (116) ou une zone d'étanchéité (117) dans la position de montage (111) pour la fermeture du tuyau d'évacuation par soufflage (54, 55),
dont l'action n'est plus présente dans la position d'application,
v.) dans la position d'application (112), l'arête frontale (59) de la partie frontale (58) libre du tuyau d'évacuation par soufflage (54, 55) dépasse d'au moins 0,5 mm pardessus le revêtement adhésif (121) du disque adhésif (110) pour un enfoncement de la peau (200),
vi.) par l'extension du disque adhésif (110) et la saillie du tuyau d'évacuation par soufflage (54, 55), la peau (200) est rendue extensible et
vii.) le disque adhésif (110) reste sur la peau (200) et peut être enlevé séparément après le retrait de l'unité cylindre-piston.

2. Unité cylindre-piston selon la revendication 1,
**caractérisée en ce**
**que** le disque adhésif (110) se trouvant dans la position de montage (111) est éloigné d'au moins un millimètre de la partie frontale avant (46) de la partie de fond (33) lui faisant face.

3. Unité cylindre-piston selon la revendication 1,
**caractérisée en ce**
**que** le disque adhésif (110) dans la position d'application (112) est adjacent à la partie frontale avant (46) de la partie de fond (33).

4. Unité cylindre-piston selon la revendication 1,
**caractérisée en ce**
**que** la paroi extérieure (57) du tuyau d'évacuation par soufflage (54, 55) est en forme de cylindre au moins sur des régions.

5. Unité cylindre-piston selon la revendication 1,
**caractérisée en ce**
**que** la partie de fond (33) présente une nervure annulaire (51) qui s'étend vers l'avant en une prolongation de la paroi externe de cylindre (32).

6. Unité cylindre-piston selon la revendication 5,
**caractérisée en ce**
**que** la nervure annulaire (51) présente une paroi interne (52) cylindrique.

7. Unité cylindre-piston selon la revendication 1,
**caractérisée en ce**
**que** le disque adhésif (110) présente deux alésages (126 et 127)) se situant l'un derrière l'autre co-axialement qui sont séparés par un joint (116) sous la forme d'une membrane.

8. Unité cylindre-piston selon la revendication 1,
**caractérisée en ce**
**que** le disque adhésif (110) est constitué de caoutchouc ou d'un élastomère ou est partiellement traité avec un silicone ou avec du téflon.

9. Unité cylindre-piston selon la revendication 5,
**caractérisée en ce**
**que** le disque adhésif (110) présente une nervure circonférentielle (123) dépassant radialement, laquelle est adjacente à l'arête interne avant (59) de la nervure annulaire (51).
